# EUROPEAN PATENT SPECIFICATION

(11) **EP 3 349 686 B1**
(45) Date of publication and mention of the grant of the patent: **12.08.2020**
(21) Application number: 16847260.3
(22) Date of filing: 15.09.2016
(51) Int. Cl.: A61F 2/00, A61F 2/44, A61F 2/30

(54) **ADJUSTABLE, IMPLANTABLE SPINAL DISC DEVICE FOR DEFORMITY CORRECTION IN INTERVERTEBRAL FUSION PROCEDURES**
VERSTELLBARE, IMPLANTIERBARE BANDSCHEIBENVORRICHTUNG ZUR DEFORMATIONSKORREKTUR IN EINGRIFFEN ZUR INTERVERTEBRALEN FUSION
DISQUE INTERVERTÉBRAL IMPLANTABLE, RÉGLABLE, POUR LA CORRECTION D'UNE MALFORMATION LORS D'INTERVENTIONS DE FUSION INTERVERTÉBRALE

(30) Priority: 18.09.2015 US 201562220301 P
(43) Date of publication of application: 25.07.2018
(73) Proprietor: The Board of Trustees of the University of Illinois, Urbana, IL 61801 (US)
(72) Inventor: NECKRYSH, Sergey, Y., Chicago, IL 60612 (US)
(74) Representative: Marles, Alan David
(86) International application number: PCT/US2016/051808
(87) International publication number: WO 2017/048886

(56) References cited:
- US-A- 5 683 465
- US-A1- 2003 171 812
- US-A1- 2004 243 240
- US-A1- 2006 015 184
- US-A1- 2008 082 173
- US-A1- 2009 164 017
- US-A1- 2011 208 312
- US-A1- 2014 236 296
- US-A1- 2014 277 501

## Description

### CROSS-REFERENCE TO A RELATED APPLICATION

This application claims the priority to U.S. Provisional Patent Application No. 62/220,301, filed September 18, 2015, inventor Sergey Neckrysh, titled "Surgical Implant Device".

### FIELD OF THE INVENTION

The present invention, in general, relates to an intervertebral implant, and more specifically, relates to an adjustable, implantable spinal disc device which can be used for deformity correction in intervertebral fusion procedures.

### BACKGROUND OF THE INVENTION

Joint degeneration and injury is a common medical condition that can occur in a variety of joints throughout the human body, especially with increasing age, and initially is often treated conservatively, such as through medication and/or physical therapy. Genetic or developmental irregularities, trauma, chronic stress, tumors, and degenerative wear are a few of the causes that can result in spinal pathologies for which surgical intervention may be necessary.

In the spine, depending upon the degree of degeneration, injury, mobility, and pain, for example, intervertebral fusion may be the surgical treatment of choice. A wide variety of systems and devices, often referred to as intervertebral "cages", have been disclosed in the art which achieve immobilization and/or fusion of adjacent bones by implanting artificial assemblies in or on the spinal column, such as for treatment of intervertebral disc degeneration, for example. The spinal intervertebral disc is situated between adjacent vertebral bodies, and comprises an annulus fibrosus and a nucleus pulposis. With respect to the failure of the intervertebral disc, for intervertebral fusion procedures, the intervertebral disc may be removed, and as a replacement, an artificial intervertebral disc device (such as an interbody fusion cage) may be inserted and secured between the adjacent vertebral bodies. Once inserted and secured, the intervertebral disc device separates and holds the adjacent vertebral bodies apart, typically relieving the pressure on affected nerves, reducing mechanical instability, and potentially also reducing corresponding pain.

With aging, however, the natural curvatures of the spine may also deteriorate, such as a straightening of the lumbar spine and loss of the natural lordosis. In addition to such anterior-posterior deformities or deteriorations, there may be additional left-right (or sagittal) deformities which have occurred over time as well. Various prior art methods of correcting such curvature deformities, however, tend to have a significantly high failure rate, with a failure to fuse on the order of 30% to 60%, for example.

As a result, a need remains for an adjustable, implantable spinal disc device which can correct such spinal deformities and degenerative changes in an intervertebral fusion procedure. In addition, such an adjustable, implantable spinal disc device should promote bony fusion with and between the adjacent vertebral bodies to achieve greater mechanical stability while simultaneously restoring appropriate spinal curvatures, such as lordosis and kyphosis. Patent document US5683465 discloses an implantable spinal disc device which features arcuate extensions of the plate members. The extensions mate and are slidably movable to each other, allowing the plate members to tilt. The central through channel of the disc device in US5683465 is not described as holding graft material.

### SUMMARY OF THE INVENTION

The representative embodiments of the present invention provide numerous advantages. Representative embodiments of an adjustable, implantable spinal disc device, as a spinal implant, assembly or cage, are disclosed having transverse plate members which each include a respective arcuate extension. The arcuate extensions have mating contours which are slidable with respect to each other (prior to fixation), generally allowing angular movement or rotation of the transverse plate members with respect to each other, in both the sagittal and coronal planes. This therefore allows the transverse plate members of the adjustable, implantable spinal disc device to be angularly offset with respect to each other, to create or restore the desired configuration of the spine *in situ*, such as a lordotic curvature or kyphotic curvature. The representative embodiments of an adjustable, implantable spinal disc device also allow mounting of additional extension or spacing endplates, in order to adjust the height (longitudinal) of the representative embodiments of an adjustable, implantable spinal disc device.

The representative embodiments of an adjustable, implantable spinal disc device further include a comparatively large and continuous central through-channel or cavity defined by the arcuate extensions, for bone graft placement and subsequent fusion of the bone graft to both of the adjacent vertebral bodies, not just one. This bone graft in the continuous central through-channel or cavity, connecting to both adjacent vertebrae, provides significantly improved mechanical stability of the fused vertebrae, and significantly reduces the graft failure rate in deformity correction. The location of the continuous central through-channel or cavity within the arcuate extensions is also especially significant, as the arcuate extensions effectively protect the bone graft material of the central through-channel or cavity from dissipation or other erosion during the healing, bone graft solidification and bone growth of the fusion process, such as protecting the bone graft material from any of the various body fluids which would otherwise contact and erode the bone graft material before the fusion process has been completed.

A representative embodiment of a spinal disc device is disclosed which is implantable between adjacent vertebral bodies, comprising: a first transverse plate member; a first arcuate extension coupled to and extending longitudinally from the first transverse plate member, the first arcuate extension having a first configuration; a second transverse plate member; and a second arcuate extension coupled to and extending longitudinally from the second transverse plate member, the second arcuate extension having a second, mating configuration with the first configuration of the first arcuate extension and slideably moveable with respect to the first arcuate extension, the first arcuate extension and the second arcuate extension further arranged to form a central through-channel between the first and second transverse plate members.

In a representative embodiment, the first transverse plate member further comprises a first through-hole and the second transverse plate member further comprises a second through-hole, the first and second through-holes arranged to communicate with the central through-channel. In a representative embodiment, the central through-channel is arranged to hold bone graft material.

In a representative embodiment, the first configuration of the first arcuate extension and the second configuration of the second arcuate extension may each be a partially spherical, convex shell configuration. For such a representative embodiment, the first arcuate extension is slideably moveable in both a sagittal plane and a coronal plane with respect to the second arcuate extension. Also in such a representative embodiment, following implantation between the adjacent vertebral bodies, the first arcuate extension may be slideably moveable in both a sagittal plane and a coronal plane with respect to the second arcuate extension for deformity correction prior to fixation.

In another representative embodiment, an inner surface of the first arcuate extension may have a partially spherical, concave configuration and an outer surface of the second arcuate extension may have a partially spherical, convex configuration; or alternatively, an inner surface of the second arcuate extension may have a partially spherical, concave configuration and an outer surface of the first arcuate extension may have a partially spherical, convex configuration.

In a representative embodiment, the first arcuate extension and the second arcuate extension may be further arranged to overlap each other in the longitudinal dimension.

In a representative embodiment, the spinal disc device also may further comprise: at least one extension endplate coupleable longitudinally to the first transverse plate member or to the second transverse plate member. For such a representative embodiment, the at least one extension endplate may further comprise a third through-hole arranged to communicate with the central through-channel.

In a representative embodiment, one or more of the first transverse plate member, the second transverse plate member, the first arcuate extension, and the second arcuate extension may comprise a material selected from the group consisting of: pyrolytic carbon, titanium, porous titanium, titanium nitride, tantalum, cobalt, chromium, polyethylene, carbon fiber, PEEK® (Polyether ether ketone), acetal homopolymer resin, alumina, zirconia, silicon carbide, silicon nitride, stainless steel, diamond, or a diamond-like material

Also in a representative embodiment, the central through-channel has a first volume and the spinal disc device has a second volume, wherein the first volume may be at least 50% of the second volume.

Representative embodiments of a kit for a spinal disc device implantable between adjacent vertebral bodies is also disclosed, with the kit comprising: a plurality of first components, each first component of the plurality of first components comprising: a first transverse plate member; and a first arcuate extension coupled to and extending longitudinally from the first transverse plate member, the first arcuate extension having a first configuration; and a plurality of second components, a second component of the plurality of second components coupleable to a first component of the plurality of first components to form a spinal disc device, each second component of the plurality of second components comprising: a second transverse plate member; and a second arcuate extension coupled to and extending longitudinally from the second transverse plate member, the second arcuate extension having a second, mating configuration with the first configuration of the first arcuate extension and slideably moveable with respect to the first arcuate extension for each of the first components, the first arcuate extension and the second arcuate extension further arranged to form a central through-channel between the first and second transverse plate members for each of the first and second components.

For a representative embodiment of such a kit, each first transverse plate member further comprises a first through-hole and each second transverse plate member further comprises a second through-hole, the first and second through-holes arranged to communicate with the central through-channel when the first component is coupled to the second component.

For a representative embodiment of such a kit, each first transverse plate member of the plurality of first components may have a different lateral dimension, or a different lateral configuration, or a different longitudinal thickness. In addition, for a representative embodiment of such a kit, each second transverse plate member of the plurality of second components has a different lateral dimension, or a different lateral configuration, or a different longitudinal thickness.

For a representative embodiment of such a kit, for each of the first and second components, the first configuration of the first arcuate extension and the second configuration of the second arcuate extension may each be a partially spherical, convex shell configuration.

Such a representative embodiment of a kit for a spinal disc device also may further comprise: a plurality of extension endplates, each extension endplate coupleable longitudinally to the first transverse plate members of the plurality of first components or to the second transverse plate members of the plurality of second components. For such a representative embodiment, each extension endplate may further comprise a third through-hole arranged to communicate with the central through-channel when coupled to the first transverse plate member or to the second transverse plate member and when the first component is coupled to the second component. Such a representative embodiment of a kit for a spinal disc device also may further comprise: a plurality of screws to couple the first transverse plate member and the second transverse plate member to adjacent vertebral bodies.

A representative embodiment of a spinal disc device is disclosed which is implantable between adjacent vertebral bodies, comprising: a first transverse plate member, the first transverse plate member further comprising a first through-hole; a first arcuate extension coupled to and extending longitudinally from the first transverse plate member, the first arcuate extension having a partially spherical, convex shell configuration; a second transverse plate member, the second transverse plate member further comprising a second through-hole; and a second arcuate extension coupled to and extending longitudinally from the second transverse plate member, the second arcuate extension having a second, mating partially spherical, convex shell configuration and slideably moveable with respect to the first arcuate extension, the first arcuate extension and the second arcuate extension further arranged to form a central through-channel between the first and second transverse plate members and in communication with the first and second through-holes.

For such a representative embodiment, the first arcuate extension and the second arcuate extension are further arranged to overlap each other in the longitudinal dimension, and wherein the central through-channel is arranged to hold bone graft material.

Numerous other advantages and features of the present invention will become readily apparent from the following detailed description of the invention and the embodiments thereof, from the claims and from the accompanying drawings.

### BRIEF DESCRIPTION OF THE DRAWINGS

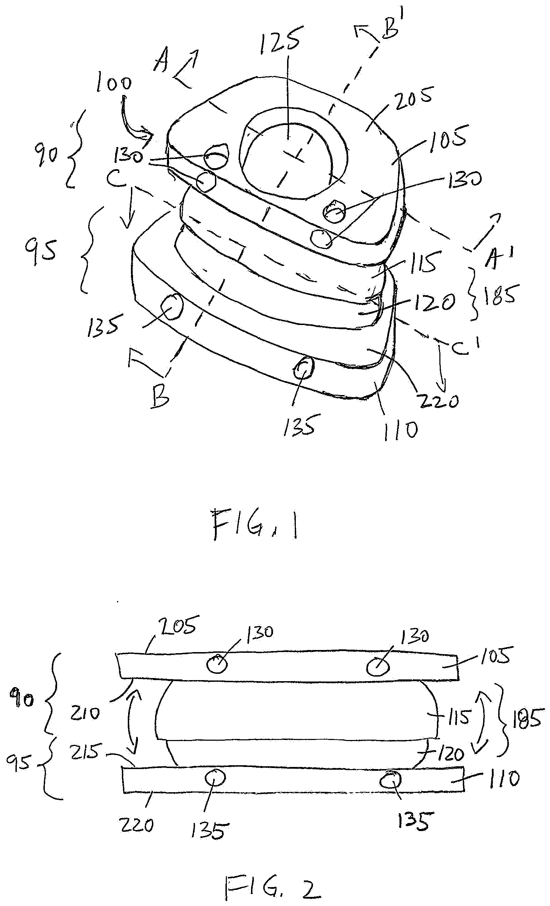

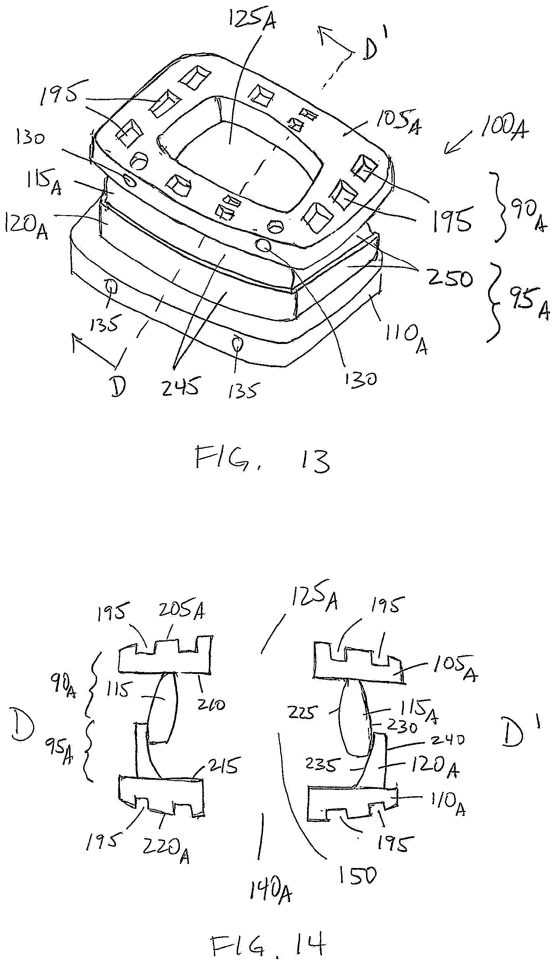

The objects, features and advantages of the present invention will be more readily appreciated upon reference to the following disclosure when considered in conjunction with the accompanying drawings, wherein like reference numerals are used to identify identical components in the various views, and wherein reference numerals with alphabetic characters are utilized to identify additional types, instantiations or variations of a selected component embodiment in the various views, in which:
Figure (or "FIG.") 1 is an isometric view of a first representative embodiment of an adjustable, implantable spinal disc device.
Figure (or "FIG.") 2 is a front elevational view of a first representative embodiment of an adjustable, implantable spinal disc device.
Figure (or "FIG.") 3 is a side elevational view of a first representative embodiment of an adjustable, implantable spinal disc device.
Figure (or "FIG.") 4 is a top, plan view of a first representative embodiment of an adjustable, implantable spinal disc device.
Figure (or "FIG.") 5 is a bottom view of a first representative embodiment of an adjustable, implantable spinal disc device.
Figure (or "FIG.") 6 is a first cross-sectional view of the first representative embodiment of an adjustable, implantable spinal disc device illustrated in FIG. 1.
Figure (or "FIG.") 7 is a second cross-sectional view of the first representative embodiment of an adjustable, implantable spinal disc device illustrated in FIG. 1.
Figure (or "FIG.") 8 is a third cross-sectional view of the first representative embodiment of an adjustable, implantable spinal disc device illustrated in FIG. 1.
Figure (or "FIG.") 9 is an isometric view of a representative embodiment of an additional, spacing endplate for use with an adjustable, implantable spinal disc device.
Figure (or "FIG.") 10 is a front elevational view of a representative embodiment an adjustable, implantable spinal disc device with additional, spacing endplates.
Figure (or "FIG.") 11 is a side elevational view of a representative embodiment of an adjustable, implantable spinal disc device illustrated between adjacent vertebral bodies and further having an anterior-posterior (coronal plane) angular adjustment.
Figure (or "FIG.") 12 is a front elevational view of a representative embodiment of an adjustable, implantable spinal disc device, with additional spacing endplates, illustrated between adjacent vertebral bodies and further having a left-right (sagittal plane) angular adjustment.
Figure (or "FIG.") 13 is an isometric view of a second representative embodiment of an adjustable, implantable spinal disc device.
Figure (or "FIG.") 14 is a cross-sectional view of a second representative embodiment of an adjustable, implantable spinal disc device illustrated in FIG. 13.
Figure (or "FIG.") 15 is an isometric view of a third representative embodiment of an adjustable, implantable spinal disc device.
Figure (or "FIG.") 16 is a top, plan view of a transverse location on a vertebral body and a corresponding potential configuration or shape of a first transverse plate member (or endplate) and/or second transverse plate member (or endplate) of a representative embodiment of an adjustable, implantable spinal disc device.
Figure (or "FIG.") 17 is an isometric view of a representative embodiment of kit for a representative embodiment of an adjustable, implantable spinal disc device.

### DETAILED DESCRIPTION OF REPRESENTATIVE EMBODIMENTS

The present invention is set out in the appended claims. However, while the present invention is susceptible of embodiment in many different forms, there are shown in the drawings and will be described herein in detail specific exemplary embodiments thereof, with the understanding that the present disclosure is to be considered as an exemplification of the principles of the invention and is not intended to limit the invention to the specific embodiments illustrated. In this respect, before explaining at least one embodiment consistent with the present invention in detail, it is to be understood that the invention is not limited in its application to the details of construction and to the arrangements of components set forth above and below, illustrated in the drawings, or as described in the examples. Apparatuses consistent with the present invention are capable of other embodiments and of being practiced and carried out in various ways. Also, it is to be understood that the phraseology and terminology employed herein, as well as the abstract included below, are for the purposes of description and should not be regarded as limiting.

Representative embodiments of an adjustable, implantable spinal disc device (100, 100_{A}, 100_{B}), as a spinal implant, assembly or cage, are disclosed having transverse plate members (or endplates) 105, 110 which each include a respective arcuate extension 115, 120 collectively forming a coupling member 185. The arcuate extensions 115, 120 have mating contours which are slidable with respect to each other (prior to fixation), generally allowing angular movement or rotation of the transverse plate members (or endplates) 105, 110 with respect to each other, in both the sagittal and coronal planes, allowing the transverse plate members (or endplates) 105, 110 of the adjustable, implantable spinal disc device (100, 100_{A}, 100_{B}) to be angularly offset with respect to each other, to create or restore the desired configuration of the spine *in situ*, such as a lordotic curvature or kyphotic curvature. The representative embodiments of an adjustable, implantable spinal disc device (100, 100_{A}, 100_{B}) further include a comparatively large and continuous central through-channel or cavity 150 defined by the arcuate extensions 115, 120, for bone graft placement and subsequent fusion of the bone graft to both of the adjacent vertebral bodies 170, 175. The representative embodiments of an adjustable, implantable spinal disc device (100, 100_{A}, 100_{B}) also allow mounting of additional extension or spacing endplates 155, in order to adjust the height (longitudinal) of the representative embodiments of an adjustable, implantable spinal disc device (100, 100_{A}, 100_{B}). The transverse plate members (or endplates) 105, 110 and any additional spacing endplates 155 also may be secured to the corresponding, adjacent vertebral bodies with one or more screws, for example.

Following removal of an intervertebral disc from between adjacent vertebral bodies, a representative embodiment of an adjustable, implantable spinal disc device (100, 100_{A}, 100_{B}) is inserted and secured between the adjacent vertebral bodies 170, 175, typically in an anterior surgical procedure. The representative embodiments of an adjustable, implantable spinal disc device (100, 100_{A}, 100_{B}) then allow changes in spine configuration in coronal and sagittal planes during the subsequent posterior stages of the surgical procedure, for deformity correction, such as to restore a lordotic curvature or a kyphotic curvature, and typically secured using rods and screws inserted into corresponding pedicles of the adjacent vertebrae. The representative embodiments of an adjustable, implantable spinal disc device (100, 100_{A}, 100_{B}) also allow for better adaptability of the adjustable, implantable spinal disc device (100, 100_{A}, 100_{B}) to the angles or the adjacent vertebral bodies during the anterior stages or the surgical procedure for other spine reconstruction and bone grafting. The representative embodiments of an adjustable, implantable spinal disc device (100, 100_{A}, 100_{B}) are suitable for cervical and lumbar applications, for example.

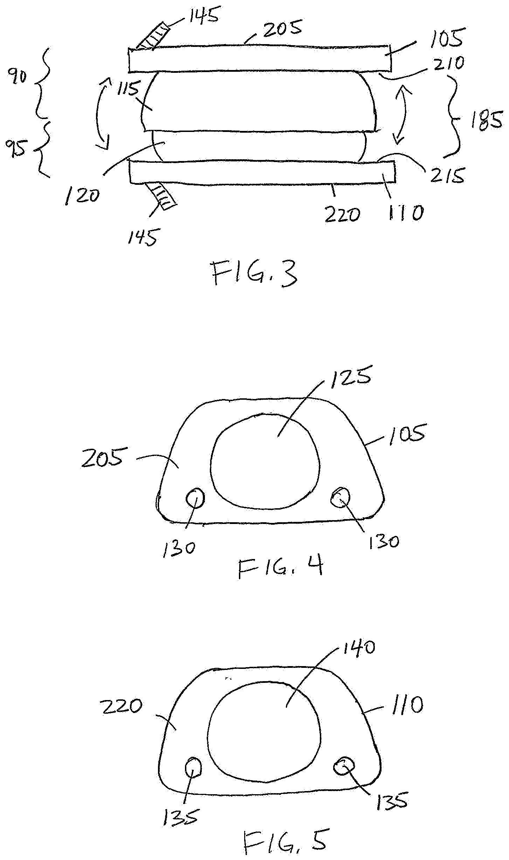

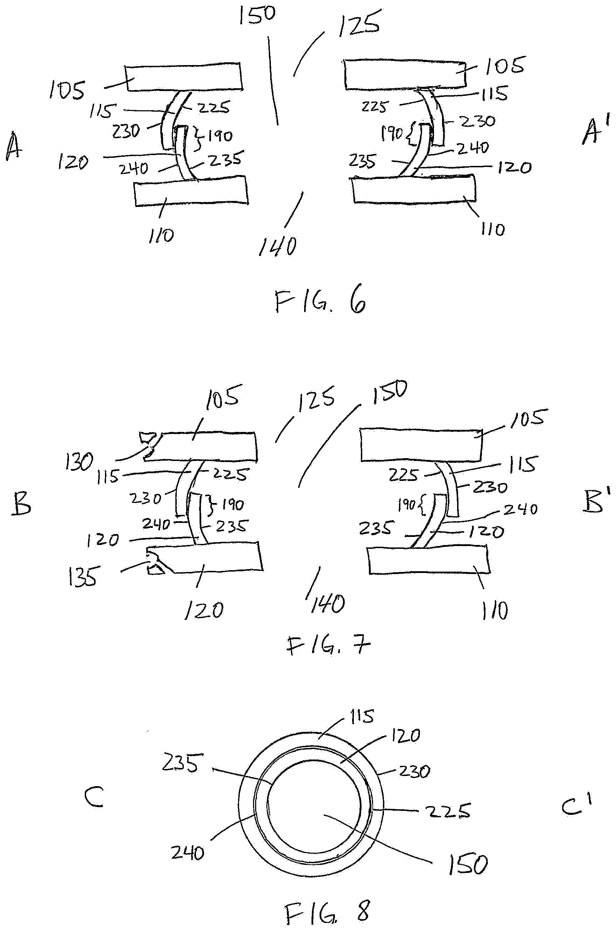

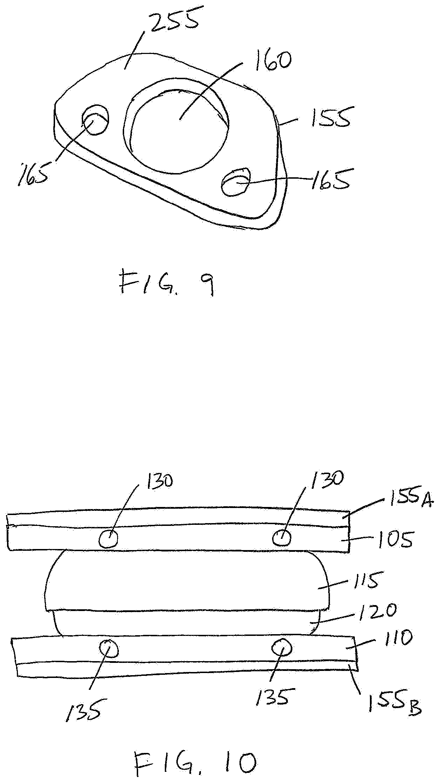

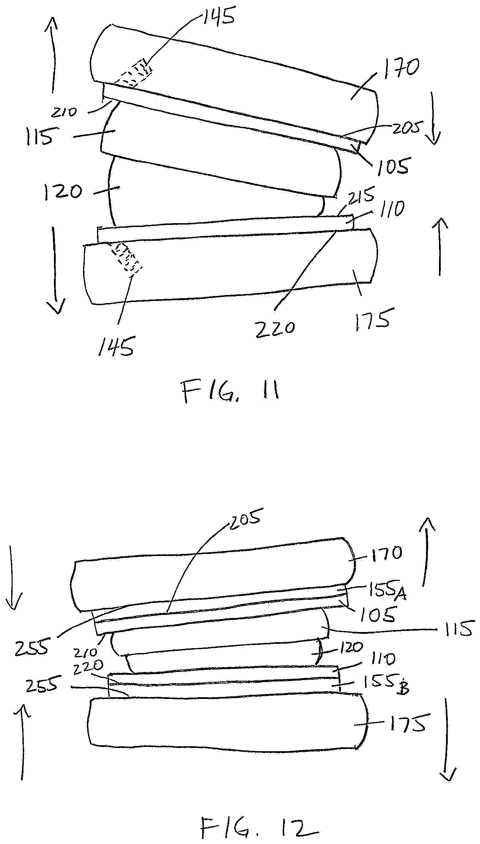

FIG. 1 is an isometric view of a first representative embodiment of an adjustable, implantable spinal disc device 100. FIG. 2 is a front elevational view of a first representative embodiment of an adjustable, implantable spinal disc device 100. FIG. 3 is a side elevational view of a first representative embodiment of an adjustable, implantable spinal disc device 100, with an added illustration (in cross-section) of screws 145 inserted in screw holes 130, 135. FIG. 4 is a top, plan view of a first representative embodiment of an adjustable, implantable spinal disc device 100. FIG. 5 is a bottom view of a first representative embodiment of an adjustable, implantable spinal disc device 100. FIG. 6 is a first cross-sectional view (through the A-A' (coronal) plane) of the first representative embodiment of an adjustable, implantable spinal disc device illustrated in FIG. 1. FIG. 7 is a second cross-sectional view (through the B-B' (sagittal) plane) of the first representative embodiment of an adjustable, implantable spinal disc device illustrated in FIG. 1, with an added illustration (in cross-section) of screw holes 130, 135. FIG. 8 is a third cross-sectional view (through the C-C' (transverse) plane) of the first representative embodiment of an adjustable, implantable spinal disc device illustrated in FIG. 1. FIG. 9 is an isometric view of a representative embodiment of an additional, spacing endplate 155 for use with an adjustable, implantable spinal disc device (100, 100_{A}, 100_{B}). FIG. 10 is a front elevational view of a first representative embodiment an adjustable, implantable spinal disc device (100, 100_{A}, 100_{B}) with additional, spacing endplates 155. FIG. 11 is a side elevational view of a representative embodiment of an adjustable, implantable spinal disc device (100, 100_{A}, 100_{B}) illustrated between adjacent vertebral bodies 170, 175 and further having an anterior-posterior (coronal plane) angular adjustment. FIG. 12 is a front elevational view of a representative embodiment of an adjustable, implantable spinal disc device (100, 100_{A}, 100_{B}), with additional spacing endplates 155, illustrated between adjacent vertebral bodies 170, 175 and further having a left-right (sagittal plane) angular adjustment.

While the various representative embodiments of an adjustable, implantable spinal disc device (100, 100_{A}, 100_{B}), for purposes of illustration and explanation, are shown and described as having different shapes and features, any of the various shapes, configurations, elements, materials and features may be utilized in any selection, in any permutation and/or combination, and in any embodiment of an adjustable, implantable spinal disc device (100, 100_{A}, 100_{B}), and any and all of which are within the scope of the claimed invention. In addition, for purposes of illustration and explanation, the directions and orientation of the representative embodiments of an adjustable, implantable spinal disc device (100, 100_{A}, 100_{B}) are discussed with reference to the coronal (anterior-posterior), sagittal (left-right) and transverse (upper-lower, or superior-inferior, or cranial- caudal) planes of a human body, with a longitudinal direction or orientation substantially perpendicular (orthogonal) to the transverse plane.

Referring to FIGs. 1 - 12, the first representative embodiment of an adjustable, implantable spinal disc device 100 comprises a first, generally transverse plate member (or endplate) 105, a second, generally transverse plate member (or endplate) 110, and a coupling member 185 extending longitudinally between and coupling the first transverse plate member (or endplate) 105 and the second transverse plate member (or endplate) 110 to each other. The first transverse plate member (or endplate) 105 has a first surface 205 and a second surface 210. The second transverse plate member (or endplate) 110 also has a first surface 215 and a second surface 220. In the first representative embodiment of an adjustable, implantable spinal disc device 100, the first and second transverse plate members (or endplates) 105, 110 are illustrated as substantially flat and having a uniform thickness, while in other embodiments discussed below, the first and second transverse plate members (or endplates) 105, 110 have other configurations, shapes, sizes, and contours which are not flat and/or not of uniform thickness.

Those having skill in the art will recognize that the first and second transverse plate members (or endplates) 105, 110 may have a plurality of configurations, shapes, sizes, and contours, any and all of which are considered equivalent and within the scope of the disclosure. In any selected embodiment of an adjustable, implantable spinal disc device (100, 100_{A}, 100_{B}), the first and second transverse plate members (or endplates) 105, 110 may have the same or similar configuration, shape, size, and contour, or the first and second transverse plate members (or endplates) 105, 110 may each have different configurations, shapes, sizes, and contours, such as for appropriate matching to different, adjacent first and second vertebral bodies 170, 175. A variety of additional configurations, shapes, sizes, and contours of the first and second transverse plate members (or endplates) 105, 110 are illustrated and discussed below with reference to FIGs. 13 - 16. In addition, any of the first and second transverse plate members (or endplates) 105, 110 may have additional surface features and/or coatings, such as additional blind or through-holes, porosity, roughness, etching, etc., as known or becomes known, any and all of which are considered equivalent and within the scope of the disclosure.

For example and without limitation, while the contact surfaces 205 and 220 may be substantially flat, such may alternatively include one or more bone adhesion facilitating elements, which are operable to promote bone adhesion to the vertebral bodies 170, 175. For example and without limitation, the bone adhesion facilitating elements may include one or more of spikes, keels, roughening elements, etc. In addition, numerous other modifications may be employed on the first and second transverse plate members (or endplates) 105, 110, such as screws, flanges, coatings, dimples, beads, shock absorption members, etc., also for example and without limitation. Additionally, the surfaces 205 and 220 of the first and second transverse plate members (or endplates) 105, 110 may further comprise at least a lateral ring of porous coating (which may be a sprayed deposition layer, an adhesive applied beaded metal layer, or other suitable porous coatings known in the art). This porous coating permits the long term ingrowth of vertebral bone into first and second transverse plate members (or endplates) 105, 110 as part of permanently securing the representative embodiments of an adjustable, implantable spinal disc device (100, 100_{A}, 100_{B}) between the adjacent vertebral bodies 170, 175. In a representative embodiment, the porous coating may comprise zirconium oxide ceramic, also for example and without limitation.

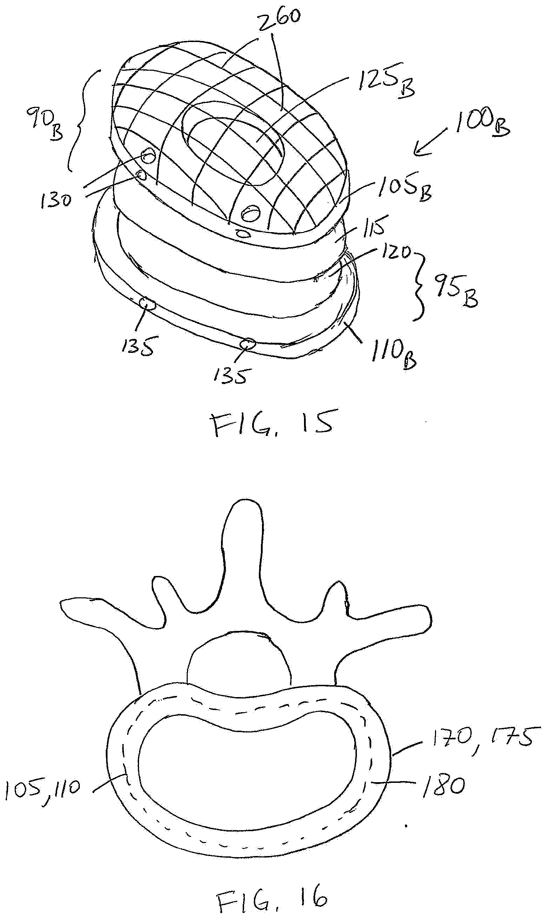

Also for example and without limitation, in any one of the representative embodiments of an adjustable, implantable spinal disc device (100, 100_{A}, 100_{B}), the first transverse plate member (or endplate) 105 and/or second transverse plate member (or endplate) 110 may be circular, elliptical, trapezoidal, rhomboidal, square, rectangular, an irregular or regular polygon or Reuleaux polygon, or any other shape or configuration in the transverse or lateral dimension or orientation. The lateral configuration or shape and size of the first and second transverse plate members (or endplates) 105, 110 are generally selected to at least partially overlap at least some of the epiphysial rim or ring (180) of the first and/or second vertebral body 170, 175, and will also generally vary with the type and size of the vertebral body 170, 175, such as a cervical or lumbar vertebral body 170, 175. Also for example and without limitation, the first and/or second transverse plate members (or endplates) 105, 110 may each be polygonal in shape or configuration in the transverse or lateral dimension or orientation, wherein the polygon comprises at least four side edges. Additionally, such a polygon may have either straight or curved sides (alternately called a Reuleaux polygon), and may also have sides with different lengths and smoothly blended intersections. For example and without limitation, as illustrated in FIGs. 1, 4, 5, and 9, the first and second transverse plate members (or endplates) 105, 110 have configurations in the transverse or lateral dimension or orientation which are generally trapezoidal polygons having curved sides with different lengths and smoothly blended intersections, while in FIG. 13, the first and second transverse plate members (or endplates) 105, 110 have configurations in the transverse or lateral dimension or orientation which are generally rectangular polygons having curved sides with different lengths and smoothly blended intersections, while in FIG. 15, the first and second transverse plate members (or endplates) 105, 110 have configurations in the transverse or lateral dimension or orientation which are generally elliptical, while in FIG. 16, the first and second transverse plate members (or endplates) 105, 110 have configurations in the transverse or lateral dimension or orientation which are generally irregular and elliptical to substantially overlap and match the epiphysial rim or ring (180) of the first and/or second vertebral body 170, 175.

When the first representative embodiment of an adjustable, implantable spinal disc device 100 is inserted in between adjacent first and second vertebral bodies 170, 175, the first surface 205 of the first transverse plate member (or endplate) 105 will be contacting or abutting the first vertebral body 170, and more particularly the epiphysial rim or ring (180) of the first vertebral body 170, and the second surface 220 of the second transverse plate member (or endplate) 110 will be contacting or abutting the second vertebral body 175, and more particularly the epiphysial rim or ring (180) of the second vertebral body 175. The first transverse plate member (or endplate) 105 and the second transverse plate member (or endplate) 110 are typically secured anteriorly to the respective adjacent first and second vertebral bodies 170, 175 using one or more screws 145 inserted into one or more (anterior) screw holes 130, 135, as shown in FIGs. 3 and 11. It also should be noted that FIG. 7 is not an exact cross-sectional view but has been modified to add and show the one or more (anterior) screw holes 130, 135 in the first transverse plate member (or endplate) 105 and the second transverse plate member (or endplate) 110, respectively. Those having skill in the art will recognize that the representative embodiments of an adjustable, implantable spinal disc device (100, 100_{A}, 100_{B}) may be secured to the respective adjacent first and second vertebral bodies 170, 175 using a wide variety of mechanisms, in addition to those illustrated, any and all of which are considered equivalent and within the scope of the disclosure. In addition, since a representative embodiment of an adjustable, implantable spinal disc device (100, 100_{A}, 100_{B}) is placed in distracted disc space wherein surrounding soft tissues are in tension, that initial screw 145 fixation may not be necessary, as long-term fixation may be provided by bone growth and grafting, discussed below.

In other embodiments discussed below, one or more extension or spacing endplates 155 may be inserted and coupled between the first transverse plate member (or endplate) 105 and the first vertebral body 170 and/or inserted and coupled between the second transverse plate member (or endplate) 110 and the second vertebral body 175.

The coupling member 185 comprises a first arcuate (or curvate) extension 115 and a second arcuate (or curvate) extension 120. The first arcuate extension 115 is coupled to and extends generally longitudinally (and in some embodiments, substantially perpendicularly) from the second surface 210 of the first transverse plate member (or endplate) 105, and the second arcuate extension 120 is coupled to and extends generally longitudinally (and in some embodiments, substantially perpendicularly) from the first surface 215 of the second transverse plate member (or endplate) 110. The first arcuate extension 115 and the second arcuate extension 120 surround and define a central through-channel or cavity 150, such that the representative embodiments of an adjustable, implantable spinal disc device 100 (and 100_{A}, 100_{B}) are substantially hollow. The lateral and longitudinal configurations of the central through-channel or cavity 150 will generally match the lateral and longitudinal configurations of the first arcuate (or curvate) extension 115 and the second arcuate (or curvate) extension 120.

The coupling member 185 can be of any design that permits articulating motion of the first arcuate (or curvate) extension 115 and a second arcuate (or curvate) extension 120 (and their respectively coupled first and second transverse plate members (or endplates) 105, 110) relative to one another, provided that the design allows for a central through-channel or cavity 150 traversing the thickness of the coupling member 185 while still maintaining sufficient strength to maintain its structural integrity under anatomical loads and stress. For example, the desired strength of the coupling member 185 can be achieved by varying the material selection and thicknesses of the first arcuate (or curvate) extension 115 and a second arcuate (or curvate) extension 120.

Also for example and without limitation, the first and second transverse plate members (or endplates) 105, 110 may be comprised of titanium or porous titanium and have a longitudinal thickness between about 1 - 10 mm, such as 2 - 3 mm, with a lateral extent and configuration selected to be an appropriate size to correspond to a selected vertebral body 170, 175. Also for example and without limitation, the first and second transverse plate members (or endplates) 105, 110 may be comprised of titanium or porous titanium and have a plate thickness between about 1 - 10 mm. Other materials which may be utilized for any of the first and second transverse plate members (or endplates) 105, 110 and the first and second arcuate (or curvate) extensions 115, 120 are discussed in greater detail below.

In addition, each of the first transverse plate member (or endplate) 105 and the second transverse plate member (or endplate) 110 have respective first and second through-holes or channels 125, 140, providing significant external access to the comparatively large and continuous central through-channel or cavity 150 defined by the first and second arcuate extensions 115, 120. These through-holes or channels 125, 140 provide for the insertion of packing of bone graft material into the central through-channel or cavity 150, to fill the central through-channel or cavity 150 and both the first and second through-holes or channels 125, 140 with the bone graft material, prior to the placement of the representative embodiment of an adjustable, implantable spinal disc device 100 (and 100_{A}, 100_{B}) between the adjacent first and second vertebral bodies 170, 175 in a surgical procedure. The central through-channel or cavity 150 and the first and second through-holes or channels 125, 140 thereby provide for a comparatively and substantially large and continuous bone graft between the adjacent first and second vertebral bodies 170, 175, further serving to mechanically stabilize and fuse the adjacent first and second vertebral bodies 170, 175.

Also for example and without limitation, in any one of the representative embodiments of an adjustable, implantable spinal disc device (100, 100_{A}, 100_{B}), the first and second through-holes or channels 125, 140 may be circular, elliptical, trapezoidal, rhomboidal, square, rectangular, an irregular or regular polygon or Reuleaux polygon, or any other shape or configuration in the transverse or lateral dimension or orientation, such as polygonal in shape or configuration in the transverse or lateral dimension or orientation, wherein the polygon comprises at least four side edges. Additionally, such a polygon may have either straight or curved sides (alternately called a Reuleaux polygon), and may also have sides with different lengths and smoothly blended intersections. For example and without limitation, as illustrated in FIGs. 1, 4, 5, and 9, the first and second through-holes or channels 125, 140 have configurations in the transverse or lateral dimension or orientation which are generally circular, while in FIG. 13, the first and second through-holes or channels 125, 140 have configurations in the transverse or lateral dimension or orientation which are generally rectangular polygons having curved sides with different lengths and smoothly blended intersections, while in FIG. 15, the first and second through-holes or channels 125, 140 have configurations in the transverse or lateral dimension or orientation which are generally elliptical.

A significant departure from the prior art, the first and second arcuate extensions 115, 120 define and surround the central through-channel or cavity 150, and effectively protect the bone graft material of the central through-channel or cavity 150 from dissipation or other erosion during the healing, bone graft solidification and bone growth of the fusion process, such as protecting the bone graft material from any of the various body fluids which would otherwise contact and erode the bone graft material before the fusion process has been completed.

As a result, in a representative embodiment, the volume of the central through-channel or cavity 150 is a significant percentage of the overall volume of the representative embodiment of an adjustable, implantable spinal disc device (100, 100_{A}, 100_{B}). For example and without limitation, the volume of the central through-channel or cavity 150 may be in the range of about 5% to 95% of the overall volume of the representative embodiment of an adjustable, implantable spinal disc device (100, 100_{A}, 100_{B}); or more particularly, the volume of the central through-channel or cavity 150 may be in the range of about 10% to 90% of the overall volume of the representative embodiment of an adjustable, implantable spinal disc device (100, 100_{A}, 100_{B}); or more particularly, the volume of the central through-channel or cavity 150 may be in the range of about 15% to 85% of the overall volume of the representative embodiment of an adjustable, implantable spinal disc device (100, 100_{A}, 100_{B}); or more particularly, the volume of the central through-channel or cavity 150 may be in the range of about 20% to 85% of the overall volume of the representative embodiment of an adjustable, implantable spinal disc device (100, 100_{A}, 100_{B}); or more particularly, the volume of the central through-channel or cavity 150 may be in the range of about 25% to 85% of the overall volume of the representative embodiment of an adjustable, implantable spinal disc device (100, 100_{A}, 100_{B}); or more particularly, the volume of the central through-channel or cavity 150 may be in the range of about 30% to 85% of the overall volume of the representative embodiment of an adjustable, implantable spinal disc device (100, 100_{A}, 100_{B}), or more particularly, the volume of the central through-channel or cavity 150 may be in the range of about 35% to 85% of the overall volume of the representative embodiment of an adjustable, implantable spinal disc device (100, 100_{A}, 100_{B}), or more particularly, the volume of the central through-channel or cavity 150 may be in the range of about 40% to 85% of the overall volume of the representative embodiment of an adjustable, implantable spinal disc device (100, 100_{A}, 100_{B}); or more particularly, the volume of the central through-channel or cavity 150 may be in the range of about 45% to 85% of the overall volume of the representative embodiment of an adjustable, implantable spinal disc device (100, 100_{A}, 100_{B}); or more particularly, the volume of the central through-channel or cavity 150 may be in the range of about 50% to 85% of the overall volume of the representative embodiment of an adjustable, implantable spinal disc device (100, 100_{A}, 100_{B}); or more particularly, the volume of the central through-channel or cavity 150 may be in the range of about 55% to 85% of the overall volume of the representative embodiment of an adjustable, implantable spinal disc device (100, 100_{A}, 100_{B}); or more particularly, the volume of the central through-channel or cavity 150 may be in the range of about 60% to 85% of the overall volume of the representative embodiment of an adjustable, implantable spinal disc device (100, 100_{A}, 100_{B}); or more particularly, the volume of the central through-channel or cavity 150 may be in the range of about 65% to 85% of the overall volume of the representative embodiment of an adjustable, implantable spinal disc device (100, 100_{A}, 100_{B}); or more particularly, the volume of the central through-channel or cavity 150 may be in the range of about 70% to 85% of the overall volume of the representative embodiment of an adjustable, implantable spinal disc device (100, 100_{A}, 100_{B}); or more particularly, the volume of the central through-channel or cavity 150 may be in the range of about 75% to 85% of the overall volume of the representative embodiment of an adjustable, implantable spinal disc device (100, 100_{A}, 100_{B}); or more particularly, the volume of the central through-channel or cavity 150 may be in the range of about 75% to 80% of the overall volume of the representative embodiment of an adjustable, implantable spinal disc device (100, 100_{A}, 100_{B}).

Stated another way, also for example and without limitation, the volume of the central through-channel or cavity 150 may be greater than about 10% of the overall volume of the representative embodiment of an adjustable, implantable spinal disc device (100, 100_{A}, 100_{B}); or more particularly, the volume of the central through-channel or cavity 150 may be greater than about 15% of the overall volume of the representative embodiment of an adjustable, implantable spinal disc device (100, 100_{A}, 100_{B}); or more particularly, the volume of the central through-channel or cavity 150 may be greater than about 20% of the overall volume of the representative embodiment of an adjustable, implantable spinal disc device (100, 100_{A}, 100_{B}); or more particularly, the volume of the central through-channel or cavity 150 may be greater than about 25% of the overall volume of the representative embodiment of an adjustable, implantable spinal disc device (100, 100_{A}, 100_{B}); or more particularly, the volume of the central through-channel or cavity 150 may be greater than about 30% of the overall volume of the representative embodiment of an adjustable, implantable spinal disc device (100, 100_{A}, 100_{B}); or more particularly, the volume of the central through-channel or cavity 150 may be greater than about 35% of the overall volume of the representative embodiment of an adjustable, implantable spinal disc device (100, 100_{A}, 100_{B}); or more particularly, the volume of the central through-channel or cavity 150 may be greater than about 40% of the overall volume of the representative embodiment of an adjustable, implantable spinal disc device (100, 100_{A}, 100_{B}); or more particularly, the volume of the central through-channel or cavity 150 may be greater than about 45% of the overall volume of the representative embodiment of an adjustable, implantable spinal disc device (100, 100_{A}, 100_{B}); or more particularly, the volume of the central through-channel or cavity 150 may be greater than about 50% of the overall volume of the representative embodiment of an adjustable, implantable spinal disc device (100, 100_{A}, 100_{B}); or more particularly, the volume of the central through-channel or cavity 150 may be greater than about 55% of the overall volume of the representative embodiment of an adjustable, implantable spinal disc device (100, 100_{A}, 100_{B}); or more particularly, the volume of the central through-channel or cavity 150 may be greater than about 60% of the overall volume of the representative embodiment of an adjustable, implantable spinal disc device (100, 100_{A}, 100_{B}); or more particularly, the volume of the central through-channel or cavity 150 may be greater than about 70% of the overall volume of the representative embodiment of an adjustable, implantable spinal disc device (100, 100_{A}, 100_{B}); or more particularly, the volume of the central through-channel or cavity 150 may be greater than about 75% of the overall volume of the representative embodiment of an adjustable, implantable spinal disc device (100, 100_{A}, 100_{B}).

In a representative embodiment, the first arcuate (or curvate) extension 115 and the second arcuate (or curvate) extension 120 are substantially solid, such as comprising plates of titanium. In another representative embodiment, the first arcuate (or curvate) extension 115 and the second arcuate (or curvate) extension 120 are substantially porous, such as comprising plates of porous titanium. In addition, any of the first and second arcuate (or curvate) extensions 115, 120 may have additional surface features and/or coatings, such as additional blind or through-holes, porosity, roughness, etching, etc., as known or becomes known, any and all of which are considered equivalent and within the scope of the disclosure, as discussed below.

For example and without limitation, each of the first arcuate (or curvate) extension 115 and the second arcuate (or curvate) extension 120 may be integrally formed, respectively, with the first transverse plate member (or endplate) 105 and the second transverse plate member (or endplate) 110, such as through casting, injection molding, stamping, 3-D printing, etc. Alternatively, also for example and without limitation, each of the first arcuate (or curvate) extension 115 and the second arcuate (or curvate) extension 120 may be formed separately and subsequently attached, respectively, to the first transverse plate member (or endplate) 105 and the second transverse plate member (or endplate) 110, such as through a laser welding process.

Those having skill in the art will also recognize that a representative embodiment of an adjustable, implantable spinal disc device (100, 100_{A}, 100_{B}) may also utilize multiple pairs of first arcuate (or curvate) extensions 115 and second arcuate (or curvate) extensions 120 (each with a central through-channel or cavity 150).

While the first arcuate (or curvate) extension 115 and the second arcuate (or curvate) extension 120 (with the central through-channel or cavity 150) are illustrated as generally centrally located on the respective first and second transverse plate members (or endplates) 105, 110, those having skill in the art will recognize that non-central locations or arrangements may also be utilized, such as more anterior or more posterior locations, or more left or more right locations, or an asymmetric location, or multiple locations, any and all of which are considered equivalent and within the scope of the disclosure.

The first arcuate extension 115 has a first, inner surface 225 facing the central through-channel or cavity 150 and a second, outer (or external) surface 230. The second arcuate extension 120 also has first, inner surface 235 facing the central through-channel or cavity 150 and a second, outer (or external) surface 240. As mentioned above, the first and second arcuate extensions 115, 120 have mating contours which are slidable with respect to each other: more specifically, the first, inner surface 225 of the first arcuate extension 115 abuts (or contacts) and has a mating contour with the second, outer surface 240 of the second arcuate extension 120, allowing the first, inner surface 225 of the first arcuate extension 115 to be slideably moveable with respect to the second, outer surface 240 of the second arcuate extension 120, and vice-versa. Depending upon the shape and configuration of the first, inner surface 225 of the first arcuate extension 115 and the second, outer surface 240 of the second arcuate extension 120, the relative movement of the first arcuate extension 115 and the second arcuate extension 120 may be rotatable and/or pivotable in the coronal plane, the sagittal plane, and/or both the coronal and sagittal planes.

As illustrated in FIGs. 6 and 7, the first and second arcuate extensions 115, 120 are illustrated as each having a substantially uniform thickness, such that the first and second surfaces 225, 230, 235, and 240 all have similar configurations, contours and/or curvatures. In addition, the first and second arcuate extensions 115, 120 are each convex and partially spherical (or partially hemispherical), each having a partially spherical and convex configuration or shape (as a section of a spherical shell) extending in the longitudinal and transverse dimensions. More particularly the first, inner surface 225 of the first arcuate extension 115 is a partially spherical (or partially hemispherical) concave surface, having a partially spherical and concave configuration or shape (as a section of a spherical surface) extending in the longitudinal and transverse dimensions, and the second, outer surface 240 of the second arcuate extension 120 is a partially spherical (or partially hemispherical) convex surface, having a partially spherical and convex configuration or shape (as a section of a spherical surface) extending in the longitudinal and transverse dimensions, to mate with the partially spherical, concave first, inner surface 225 of the first arcuate extension 115. With such a partially spherical configuration, the first arcuate extension 115 and the second arcuate extension 120 are slideably moveable, with respect to each other, in both the coronal and sagittal planes, providing an articulating motion, to correspondingly provide for deformity correction in both the coronal and sagittal planes. As used herein, "articulation" and "articulating motion" refer to articulation (such as orientations of the first and second transverse plate members (or endplates) 105, 110 that would result from anterior-posterior flexing and lateral bending of the vertebral bones), rotation, translation, and/or any combination of the above motions. In other embodiments discussed below, the first and second arcuate extensions 115, 120 have different configurations, shapes and contours, such as for slidable movement in only the coronal (anterior-posterior) plane.

Depending upon the selected embodiment, the first and second arcuate extensions 115 also overlap each other longitudinally, illustrated as overlapping regions 190. In a representative embodiment, the first and second arcuate extensions 115 are sized and shaped to continue to overlap each other throughout the entire range of articulating motion, to continue to surround the central through-channel or cavity 150 without significant interruption or external exposure of the bone graft material within the central through-channel or cavity 150, in order to maintain any bone graft material within the central through-channel or cavity 150.

FIG. 11 is a side elevational view of a representative embodiment of an adjustable, implantable spinal disc device (100, 100_{A}, 100_{B}) illustrated between adjacent vertebral bodies 170, 175 and further having an anterior-posterior (coronal plane) angular adjustment. As illustrated in FIG. 11, the first, inner surface 225 of the first arcuate extension 115 and the second, outer surface 240 of the second arcuate extension 120 are each partially spherical surfaces, providing for the first and second arcuate extensions 115, 120 to rotate anteriorly and posteriorly (in the coronal plane) with respect to each other, moving the posterior portions of the first and second transverse plate members (or endplates) 105, 110 closer to each other and moving the anterior portions of the first and second transverse plate members (or endplates) 105, 110 farther away from each other, providing a lordotic contour, as illustrated. Not separately illustrated, conversely, the first and second arcuate extensions 115, 120 alternatively may be rotated anteriorly and posteriorly (in the coronal plane) with respect to each other, moving the posterior portions of the first and second transverse plate members (or endplates) 105, 110 farther away from each other and moving the anterior portions of the first and second transverse plate members (or endplates) 105, 110 closer to each other, providing a kyphotic contour.

FIG. 12 is a front elevational view of a representative embodiment of an adjustable, implantable spinal disc device (100, 100_{A}, 100_{B}), with additional spacing endplates 155, illustrated between adjacent vertebral bodies 170, 175 and further having a left-right (sagittal plane) angular adjustment. As illustrated in FIG. 12, the first, inner surface 225 of the first arcuate extension 115 and the second, outer surface 240 of the second arcuate extension 120 are also each partially spherical surfaces, providing for the first and second arcuate extensions 115, 120 to rotate left and right (in the sagittal plane) with respect to each other, moving the left-side portions of the first and second transverse plate members (or endplates) 105, 110 closer to each other and moving the right-side portions of the first and second transverse plate members (or endplates) 105, 110 farther away from each other, as illustrated, or vice-versa, such as to provide some correction of a scoliosis deformity. In a representative embodiment, in which rods and screws are utilized bilaterally to secure the intervertebral fusion in the posterior portion of the surgical procedure, this compression of one side in the sagittal plane may occur when the rods are placed initially on one (left) side, then straightened when the rods are placed on the other (right) side, eliminating any rotation left and right (in the sagittal plane) in the final positioning of the representative embodiments of an adjustable, implantable spinal disc device (100, 100_{A}, 100_{B}).

Referring to FIGs. 9 and 10, an extension or spacing endplate 155 is illustrated, and is used to adjust the height (longitudinally) of the representative embodiments of an adjustable, implantable spinal disc device (100, 100_{A}, 100_{B}). Such an extension or spacing endplate 155 is generally added or attached to the first surface 205 of a first transverse plate member (or endplate) 105, or to the second surface 220 of a second transverse plate member (or endplate) 110, or multiple extension or spacing endplates 155 may be utilized, illustrated as a first extension or spacing endplate 155_{A} and a second extension or spacing endplate 155_{B} in FIG. 12. In addition, multiple extension or spacing endplates 155 may also be stacked with each other, and also added or attached to the first surface 205 of a first transverse plate member (or endplate) 105, or to the second surface 220 of a second transverse plate member (or endplate) 110. Such a representative extension or spacing endplate 155 is generally configured or shaped transversely similarly to the corresponding first and/or second transverse plate members (or endplates) 105, 110, and also comprises a central through-hole 160, to provide continuous access into the central through-channel or cavity 150, and typically also comprises one or more anterior screw holes 165, to be secured with the corresponding first and/or second transverse plate members (or endplates) 105, 110 into the corresponding vertebral body 170, 175, as illustrated in FIG. 12. Other types of attachment may also be utilized, such as various biological adhesives.

A representative extension or spacing endplate 155 has a third vertebral body contact surface 255 and may have any of a plurality of thicknesses (longitudinally), to provide adjustable and/or customizable spacing and sizing of the representative embodiments of an adjustable, implantable spinal disc device (100, 100_{A}, 100_{B}) for insertion between adjacent vertebral bodies 170, 175. Those having skill in the art will recognize that the extension or spacing endplate 155 may have a plurality of configurations, shapes, sizes, and contours as discussed above for the first and/or second transverse plate members (or endplates) 105, 110, any and all of which are considered equivalent and within the scope of the disclosure. In addition, a representative extension or spacing endplate 155 may have additional surface features and/or coatings, as discussed above and below, as known or becomes known, any and all of which are considered equivalent and within the scope of the disclosure.

Also for example and without limitation, the representative extension or spacing endplate 155 may be comprised of titanium or porous titanium and have a longitudinal thickness between about 0.5 - 10 mm, with a lateral extent and configuration selected to be an appropriate size to correspond to a selected vertebral body 170, 175.

FIG. 13 is an isometric view of a second representative embodiment of an adjustable, implantable spinal disc device 100_{A}, and serves to illustrate several available variations, any and all of which may be used singularly or in combination in any of the various embodiments. FIG. 14 is a cross-sectional view (through the D-D' (sagittal) plane) of the second representative embodiment of an adjustable, implantable spinal disc device 100_{A} illustrated in FIG. 13. As illustrated in FIGs. 13 and 14, the first and second arcuate extensions 115_{A}, 120_{A} overlap each other in an opposite orientation compared to the first representative embodiment of an adjustable, implantable spinal disc device 100. For this second representative embodiment of an adjustable, implantable spinal disc device 100_{A}, the first and second arcuate extensions 115_{A}, 120_{A} have convex curvatures on anterior and posterior sides 245, but are substantially flat on the lateral sides 250, such that the first and second arcuate extensions 115_{A}, 120_{A} have mating configurations or contours which are slideably moveable anteriorly and posteriorly (in the coronal plane) with respect to each other, but not left and right in the sagittal plane. For this representative embodiment, the second, outer surface 230 of the first arcuate extension 115_{A} (of the anterior and posterior sides 245) abuts (or contacts) and has a mating (convex) configuration or contour with the first, inner (concave) surface 235 of the second arcuate extension 120_{A} (of the anterior and posterior sides 245), allowing the first, inner surface 235 of the second arcuate extension 120_{A} to be slideably moveable anteriorly and posteriorly (in the coronal plane) with respect to the second, outer surface 230 of the first arcuate extension 115_{A}, and vice-versa.

Also as illustrated for the second representative embodiment of an adjustable, implantable spinal disc device 100_{A}, the first and second arcuate extensions 115_{A}, 120_{A} do not have substantially uniform thicknesses and are not necessarily convex or concave (*e.g*., the second, outer surface 240 of the second arcuate extension 120_{A} (of the anterior and posterior sides 245) is substantially rectilinear or straight and extends substantially perpendicularly from the second transverse plate member (or endplate) 110). In addition, the first, inner surface 225 of the first arcuate extension 115_{A} and the second, outer surface 240 of the second arcuate extension 120_{A} do not have mating contours with respect to each other. In addition, for the lateral sides 250, the first and second arcuate extensions 115_{A}, 120_{A} are substantially rectilinear, without convex or concave curvatures.

Also as illustrated in FIGs. 13 and 14, the first and second transverse plate members (or endplates) 105_{A}, 110_{A} have configurations in the transverse or lateral dimension or orientation which are generally rectangular polygons having curved sides with different lengths and smoothly blended intersections. In addition, the first surface 205_{A} of the first transverse plate member (or endplate) 105_{A} and the second surface 220_{A} of the second transverse plate member (or endplate) 110_{A} are dome-shaped, rather than substantially flat, such as to match the contour of the corresponding vertebral body 170, 175. The first surface 205_{A} of the first transverse plate member (or endplate) 105_{A} and the second surface 220_{A} of the second transverse plate member (or endplate) 110_{A} are also illustrated as having surface features, such as cavities 195, such as for placement or adherence of additional bone graft material.

FIG. 15 is an isometric view of a third representative embodiment of an adjustable, implantable spinal disc device 100_{B}, and illustrates several additional variations and features. As illustrated, the first transverse plate member (or endplate) 105 and the second transverse plate member (or endplate) 110 are each substantially elliptical in the lateral dimension. In addition, the first and second through-holes or channels 125, 140 are also substantially elliptical in the lateral dimension. The first arcuate (or curvate) extension 115 and the second arcuate (or curvate) extension 120 are each substantially partially spherical and convex, as described above for the first representative embodiment of an adjustable, implantable spinal disc device 100.

As illustrated in FIG. 15, one or both of first and second transverse plate members (or endplates) 105, 110 may further comprise a convex metal mesh 260 that is attached, respectively, to the surfaces 205 and 220 of the first and second transverse plate members (or endplates) 105_{B}, 110_{B}, which may have any configuration or size, such as to substantially match the configuration and size of the first and second transverse plate members (or endplates) 105_{B}, 110_{B}. Such a convex metal mesh 260 may be secured at its perimeter, for example by laser welds, to the surfaces 205 and 220 of the first and second transverse plate members (or endplates) 105_{B}, 110_{B}. Such a convex metal mesh 260 may be dome-shaped in its initial undeflected conformation, but deflects as necessary during insertion of a representative embodiment of the adjustable, implantable spinal disc device 100_{B} between vertebral bodies 170, 175, and, once the adjustable, implantable spinal disc device 100_{B} is seated between the vertebral bodies 170, 175, deforms as necessary under anatomical loads to reshape itself to the concave surface of the vertebral body 170, 175 endplate(s). This may afford the first and second transverse plate members (or endplates) 105_{B}, 110_{B} having the metal mesh substantially superior gripping and holding strength upon initial implantation. The convex metal mesh 260 further provides an osteoconductive surface through which the bone may ultimately grow. The convex metal mesh 260 may be comprised of titanium, but can also be formed from any of the other metals and/or non-metals discussed below without departing from the scope of the present invention.

FIG. 16 is a top, plan view of a transverse location on a vertebral body 170, 175 and a corresponding potential configuration or shape (illustrated in dashed lines) of a first transverse plate member (or endplate) 105 and/or second transverse plate member (or endplate) 110 of a representative embodiment of an adjustable, implantable spinal disc device (100, 100_{A}, 100_{B}). In addition to any of the circular, elliptical, trapezoidal, rhomboidal, square, rectangular, an irregular or regular polygons or Reuleaux polygons discussed above, such a first and/or second transverse plate member (or endplate) 105, 110 may generally have any shape, such as the illustrated irregular elliptical configuration. In a representative embodiment, as mentioned above, the first and/or second transverse plate member (or endplate) 105, 110 are generally configured laterally to have at least some contact with the epiphysial rim or ring (180) of the first and/or second vertebral body 170, 175, respectively.

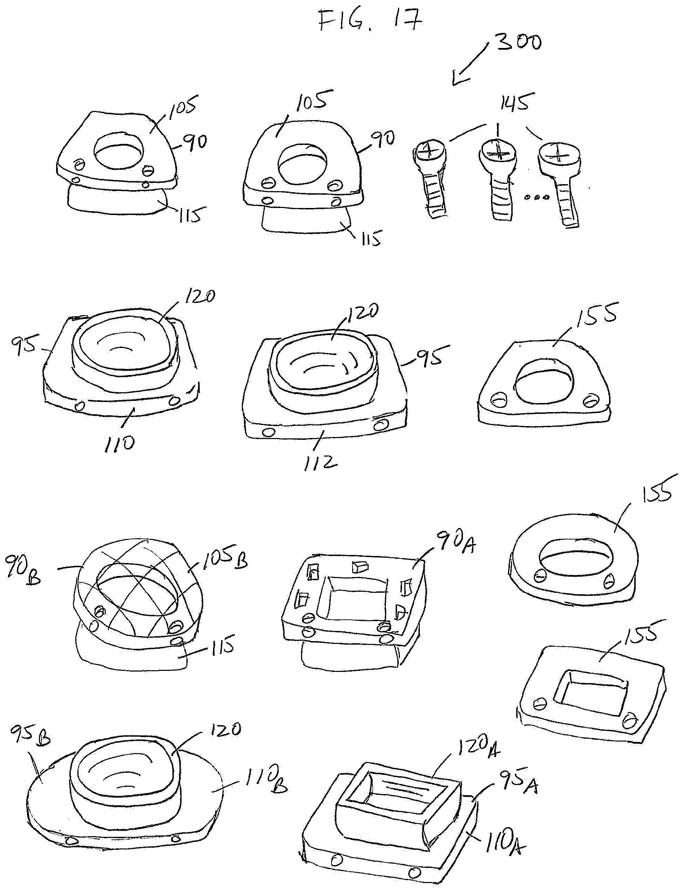

FIG. 17 is an isometric view of a representative embodiment of kit 300 for a representative embodiment of an adjustable, implantable spinal disc device (100, 100_{A}, 100_{B}). The various representative embodiments of an adjustable, implantable spinal disc device (100, 100_{A}, 100_{B}) may be provided as two separate components, a first component 90 comprising a first transverse plate member (or endplate) 105 coupled to a first arcuate (or curvate) extension 115 and a second component 95 comprising a second transverse plate member (or endplate) 110 coupled to a second arcuate (or curvate) extension 120. As should be readily apparent, the substantially two-piece construction of the various representative embodiments of an adjustable, implantable spinal disc device (100, 100_{A}, 100_{B}) permits a surgeon to select, mix, and match the first component 90 comprising a first transverse plate member (or endplate) 105 coupled to a first arcuate (or curvate) extension 115, the second component 95 comprising a second transverse plate member (or endplate) 110 coupled to a second arcuate (or curvate) extension 120, and/or extension or spacing endplates 155, before or during the surgical procedure. This may accommodate the particular anatomy of the patient, such as differing sized vertebrae, etc. Moreover, once the surgeon has selected the first and second components 90, 95, they may be easily coupled together and implanted as one singular adjustable, implantable spinal disc device (100, 100_{A}, 100_{B}).

Accordingly, in another embodiment, the present disclosure includes a kit 300 comprising: (i) one or more first components comprising a first transverse plate member (or endplate) 105 coupled to a first arcuate (or curvate) extension 115; and (ii) one or more second components comprising a second transverse plate member (or endplate) 110 coupled to a second arcuate (or curvate) extension 120, wherein the first arcuate (or curvate) extension 115 and the second arcuate (or curvate) extension 120 have mating surfaces, as discussed above, to permitting an articulating motion (in the coronal and/or sagittal planes) of the first arcuate (or curvate) extension 115 and the second arcuate (or curvate) extension 120 with respect to each other.

In another embodiment, the kit 300 further comprises one or more fixation screws 145 for attaching the first and second transverse plate members (or endplates) 105, 110 to the corresponding adjacent vertebral bodies 170, 175.

In another embodiment, the kit 300 further comprises one or more stackable extension or spacing endplates 155 having a third vertebral body contact surface 255 and outside dimensions substantially corresponding to the outside dimensions of the first transverse plate member (or endplate) 105 and/or second transverse plate member (or endplate) 110.

As mentioned above, the various representative embodiments of an adjustable, implantable spinal disc device (100, 100_{A}, 100_{B}), for purposes of illustration and explanation, are shown and described as having different shapes and features, any of the various shapes, configurations, elements, materials and features may be utilized in any selection, in any combination, and in any embodiment of an adjustable, implantable spinal disc device (100, 100_{A}, 100_{B}), and any and all of which selections and combinations are within the scope of the claimed invention.

The first and second transverse plate members (or endplates) 105, 110 and the first and second arcuate (or curvate) extensions 115, 120 may be constructed of any biocompatible material having sufficient strength to avoid breaking or deforming under the expected anatomical stress or loading. In any one of the embodiments described herein, the first and second transverse plate members (or endplates) 105, 110 and the first and second arcuate (or curvate) extensions 115, 120, in any combination or selection, may comprise or be manufactured from at least one of the following materials, for example and without limitation: pyrolytic carbon, titanium, porous titanium, titanium nitride, tantalum, cobalt, chromium, polyethylene, carbon fiber, PEEK® (Polyether ether ketone), Delrin® (acetal homopolymer resin), alumina, zirconia, silicon carbide, silicon nitride, stainless steel, diamond, or a diamond-like material. In certain embodiments, for example and without limitation, the first and second transverse plate members (or endplates) 105, 110 and the first and second arcuate (or curvate) extensions 115, 120 may be constructed of chrome, cobalt or titanium.

In addition, any of the various surfaces of the first and second transverse plate members (or endplates) 105, 110 and the first and second arcuate (or curvate) extensions 115, 120 may have one or more surface treatments, such as comprising a roughened, coated or porous surface. Surface roughness, (such as grit blasted, textured, porous, laser sintered, roughened porous spray titanium or as coated pyrolytic carbon); coatings (such as Hydroxyapatite); porous coatings (such as porous titanium, tantalum or silicon nitride); or porous formation (such as chemical etching of the surface); on such surface(s) are meant to promote fibrous or bony on-growth and/or ingrowth as a means of further anchoring the representative embodiment of an adjustable, implantable spinal disc device (100, 100_{A}, 100_{B}). These surface treatments are those commonly known to the industry and one skilled in the art.

In any one of the embodiments described herein, one or more of the surfaces of the first and second transverse plate members (or endplates) 105, 110 and the first and second arcuate (or curvate) extensions 115, 120 comprise a textured surface, wherein the textured surface is a roughened surface configured to receive a fixation compound. Such surfaces may be machined textured, laser finished textured surfaces, chemically treated (*i.e*., acid etched), or comprise a metallurgically applied coating. In some representative embodiments of an adjustable, implantable spinal disc device (100, 100_{A}, 100_{B}), one or more surfaces may comprise a textured surface, wherein the textured surface is a porous coating. In some embodiments, the porous coating is intended to replicate the pore structure of cancellous bone. Typical materials for textured and porous coated surfaces include: CPTi, CoCr beads, tantalum, porous PEEK, etc. In general, a coating can be configured from any chemically compatible material that will securely bond to the base material of the representative embodiment of an adjustable, implantable spinal disc device (100, 100_{A}, 100_{B}). Alternatively, a non-articulating surface may comprise one or more fenestrations, wherein a fenestrated surface is a surface configured to receive a fixation compound, for example and without limitation.

As defined herein, a fixation compound may comprise one or more biologic or polymerizing cements. Biologic examples include morselized bone graph or paste, or any comparable bone-graft-substitute material, cells, proteins, biologic glue, tissue sealants and fibrin sealants. Examples of polymerizing cement include polymethyl methacrylate (PMMA or Plexi-glas), glue, cement, epoxy, bonding agent, fixative, paste, adhesive, adherent, binding agent, sealant, mortar, grout or any compatible synthetic, self-curing organic or inorganic material used to fill up a cavity or to create a mechanical fixation. Alternatively, the fixation compound may comprise a combination of any one of the aforementioned biologic and polymerizing cements. Fixation compounds may be used to permanently fix a representative embodiment of an adjustable, implantable spinal disc device (100, 100_{A}, 100_{B}) to a surface of a vertebral body 170, 175; or alternately may be used to permanently bond assembled (implant) components together.

Bone grafts may be autologous or autogenous (bone harvested from the patient's own body, often from the iliac crest), allograft (cadaveric bone usually obtained from a bone bank), synthetic (often made of hydroxyapatite or other naturally occurring and biocompatible substances) with similar mechanical properties to bone, and bone morphogenic compounds and proteins. For example, demineralized bone matrix (DBM) is an allograft material devoid of mineral phase, leaving behind the organic phase comprising of an osteoconductive composite matrix of collagen and non-collagenous proteins. DBM with varying bone morphogenetic protein (BMP) content are available from the following manufacturers, for example and without limitation: Grafton (Osteotech, New Jersey), musculoskeletal transplant foundation (MTF) (Synthes, Pennsylvania), and AlloMatrix (Wright Medical, Tennessee). Also for example, ceramics may be utilized for bone grafts, such as calcium sulfate [hydroxyapatite (HA) and tricalcium phosphate], bovine collagen, natural coral, calcium carbonate, or a combination of these.

Any type or kind of bone graft material may be utilized including, for example and without limitation: synthetic bone graft materials such as Actifuse®, Novabone®, chronOS™, Formagraft®, Mozaik™, Vitoss® MASTERGRAFT® Family; traditional and nontraditional allograft materials such as mineralized tissue, biocomposites (e.g., PLEXUR P® Biocomposite, PLEXUR M® Biocomposite), and demineralized tissue (e.g., Accell®, DBX®, Osteocel® Plus, PROGENIX® Family, GRAFTON® Family, MAGNIFUSE® Family, Trinity® Evolution™); and bone morphogenic compounds and proteins, including recombinant BMP-2 (rhBMP-2) along with recombinant BMP-7 (osteogenic protein-1, OP-1) and autologous growth factor concentrate (AGF) (e.g., rhBMP, rhBMP-7, rhBMP-2, OP-1® Putty/Implant, INFUSE® Bone Graft); and synthetic scaffolds and polymers, including hydrogels.

The representative embodiments of an adjustable, implantable spinal disc device (100, 100_{A}, 100_{B}) may be implanted using methods known to one of skill in the art. Representative surgical techniques are described in Edward C. Benzel, Spine Surgery Techniques, Complication Avoidance and Management, 3rd Ed. (Saunders, 2012); and in "Femoral Cortical Ring Plus Cancellous Dowel: An Alternative in an Interior Lumbar Interbody Fusion" available from Richard M. Salib, M.D., Institute for Low Back Care, 2800 Chicago Avenue South, Minneapolis, Minn. 55407.

Not separately illustrated, various inserting devices may be utilized in the surgical procedures to hold the first and second components 90, 95 together, with bone graft material within the central through-channel or cavity 150, and then implant a representative embodiment of an adjustable, implantable spinal disc device (100, 100_{A}, 100_{B}). In addition, also not separately illustrated, the representative embodiments of an adjustable, implantable spinal disc device (100, 100_{A}, 100_{B}) may have additional elements or features for use with such an inserting device, such as coupling holes or slots, for example and without limitation.

The present disclosure is to be considered as an exemplification of the principles of the invention and is not intended to limit the invention to the specific embodiments illustrated. In this respect, it is to be understood that the invention is not limited in its application to the details of construction and to the arrangements of components set forth above and below, illustrated in the drawings, or as described in the examples. Systems, methods and apparatuses consistent with the present invention are capable of other embodiments and of being practiced and carried out in various ways.

Although the invention has been described with respect to specific embodiments thereof, these embodiments are merely illustrative and not restrictive of the invention. In the description herein, numerous specific details are provided, such as examples of electronic components, electronic and structural connections, materials, and structural variations, to provide a thorough understanding of embodiments of the present invention. One skilled in the relevant art will recognize, however, that an embodiment of the invention can be practiced without one or more of the specific details, or with other apparatus, systems, assemblies, components, materials, parts, etc. In other instances, well-known structures, materials, or operations are not specifically shown or described in detail to avoid obscuring aspects of embodiments of the present invention. In addition, the various Figures are not drawn to scale and should not be regarded as limiting.

Reference throughout this specification to "one embodiment", "an embodiment", or a specific "embodiment" means that a particular feature, structure, or characteristic described in connection with the embodiment is included in at least one embodiment of the present invention and not necessarily in all embodiments, and further, are not necessarily referring to the same embodiment. Furthermore, the particular features, structures, or characteristics of any specific embodiment of the present invention may be combined in any suitable manner and in any suitable combination with one or more other embodiments, including the use of selected features without corresponding use of other features. In addition, many modifications may be made to adapt a particular application, situation or material to the essential scope und of the present invention. It is to be understood that other variations and modifications of the embodiments of the present invention described and illustrated herein are possible in light of the teachings herein and are to be considered part of the scope of the present invention.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated. In addition, every intervening sub-range within range is contemplated, in any combination, and is within the scope of the disclosure. For example, for the range of 5 - 10, the sub-ranges 5 - 6, 5 - 7, 5 - 8, 5 - 9, 6 - 7, 6 - 8, 6 - 9, 6 - 10, 7 - 8, 7 - 9, 7 - 10, 8 - 9, 8 - 10, and 9 - 10 are contemplated and within the scope of the disclosed range.

It will also be appreciated that one or more of the elements depicted in the Figures can also be implemented in a more separate or integrated manner, or even removed or rendered inoperable in certain cases, as may be useful in accordance with a particular application. Integrally formed combinations of components are also within the scope of the invention, particularly for embodiments in which a separation or combination of discrete components is unclear or indiscernible. In addition, use of the term "coupled" herein, including in its various forms such as "coupling" or "couplable", means and includes any direct or indirect electrical, structural or magnetic coupling, connection or attachment, or adaptation or capability for such a direct or indirect electrical, structural or magnetic coupling, connection or attachment, including integrally formed components and components which are coupled via or through another component.

Furthermore, any signal arrows in the drawings/Figures should be considered only exemplary, and not limiting, unless otherwise specifically noted. Combinations of components of steps will also be considered within the scope of the present invention, particularly where the ability to separate or combine is unclear or foreseeable. The disjunctive term "or", as used herein and throughout the claims that follow, is generally intended to mean "and/or", having both conjunctive and disjunctive meanings (and is not confined to an "exclusive or" meaning), unless otherwise indicated. As used in the description herein and throughout the claims that follow, "a", "an", and "the" include plural references unless the context clearly dictates otherwise. Also as used in the description herein and throughout the claims that follow, the meaning of "in" includes "in" and "on" unless the context clearly dictates otherwise.

The foregoing description of illustrated embodiments of the present invention, including what is described in the summary or in the abstract, is not intended to be exhaustive or to limit the invention to the precise forms disclosed herein. From the foregoing, it will be observed that numerous variations, modifications and substitutions are intended and may be effected without departing from the scope of the novel concept of the invention. It is to be understood that no limitation with respect to the specific methods and apparatus illustrated herein is intended or should be inferred. It is, of course, intended to cover by the appended claims all such modifications as fall within the scope of the claims.

## Claims

1. A spinal disc device (100, 100_{A}, 100_{B}) implantable between adjacent vertebral bodies (170, 175), comprising:
a first transverse plate member (105);
a first arcuate extension (115) coupled to and extending longitudinally from the first transverse plate member (105), the first arcuate extension (115) having a first configuration;
a second transverse plate member (110);
a second arcuate extension (120) coupled to and extending longitudinally from the second transverse plate member (110), the second arcuate extension (120) having a second, mating configuration with the first configuration of the first arcuate extension (115) and slideably moveable with respect to the first arcuate extension (115), the first arcuate extension (115) and the second arcuate extension (120) further arranged to form a central through-channel (150) between the first and second transverse plate members (105, 110); and
wherein the central through-channel (150) is arranged to hold bone graft material,
wherein a posterior portion of each of the first and second transverse plate members is movable closer to or farther from one another as an anterior portion of each of the first and second transverse plate members respectively move farther from or closer to one another.

2. The spinal disc device (100, 100_{A}, 100_{B}) of claim 1, wherein the first transverse plate member (105) further comprises a first through-hole (125) and the second transverse plate member (110) further comprises a second through-hole (140), the first and second through-holes (125, 140) arranged to communicate with the central through-channel (150).

3. The spinal disc device (100, 100_{A}, 100_{B}) of claim 1, wherein the first configuration of the first arcuate extension (115) and the second configuration of the second arcuate extension (120) are each a partially spherical, convex shell configuration.

4. The spinal disc device (100, 100_{A}, 100_{B}) of claim 3, wherein the first arcuate extension (115) is slideably moveable in both a sagittal plane and a coronal plane with respect to the second arcuate extension (120).

5. The spinal disc device (100, 100_{A}, 100_{B}) of any of claims 1-4, wherein an inner surface (225) of the first arcuate extension (115) has a partially spherical, concave configuration and an outer surface of the second arcuate extension (120) has a partially spherical, convex configuration, or wherein an inner surface (235) of the second arcuate extension (120) has a partially spherical, concave configuration and an outer surface of the first arcuate extension (115) has a partially spherical, convex configuration.

6. The spinal disc device (100, 100_{A}, 100_{B}) of any of claims 1-5, wherein the first arcuate extension (115) and the second arcuate extension (120) are further arranged to overlap each other in the longitudinal dimension.

7. The spinal disc device (100, 100_{A}, 100_{B}) of any of claims 1-6, further comprising:
at least one extension endplate coupleable longitudinally to the first transverse plate member (105) or to the second transverse plate member (110).

8. The spinal disc device of claim 7, wherein the at least one extension endplate further comprises a third through-hole arranged to communicate with the central through-channel (150).

9. The implantable spinal disc device (100, 100_{A}, 100_{B}) of claim 1, wherein one or more of the first transverse plate member (105), the second transverse plate member (110), the first arcuate extension (115), and the second arcuate extension (120) comprise a material selected from the group consisting of: pyrolytic carbon, titanium, porous titanium, titanium nitride, tantalum, cobalt, chromium, polyethylene, carbon fiber, PEEK® (Polyether ether ketone), acetal homopolymer resin, alumina, zirconia, silicon carbide, silicon nitride, stainless steel, diamond, or a diamond-like material

10. The implantable spinal disc device (100, 100_{A}, 100_{B}) of claim 1, wherein the central through-channel (150) has a first volume and the spinal disc device has a second volume, wherein the first volume is at least 50% of the second volume.

11. A kit (300) for a spinal disc device (100, 100_{A}, 100_{B}) implantable between adjacent vertebral bodies, comprising:
a plurality of first components (90), each first component (90) of the plurality of first components (90) comprising:
a first transverse plate member (105); and
a first arcuate extension (115) coupled to and extending longitudinally from the first transverse plate member (105), the first arcuate extension (115) having a first configuration;
and
a plurality of second components (95), a second component of the plurality of second components coupleable to a first component of the plurality of first components to form a spinal disc device, each second component (95) of the plurality of second components (95) comprising:
a second transverse plate member (110);
a second arcuate extension (120) coupled to and extending longitudinally from the second transverse plate member (110), the second arcuate extension (120) having a second, mating configuration with the first configuration of the first arcuate extension (115) and slideably moveable with respect to the first arcuate extension (115) for each of the first components (90), the first arcuate extension (115) and the second arcuate extension (120) further arranged to form a central through-channel (150) between the first and second transverse plate members (105, 110) for each of the first and second components (90, 95); and
wherein the central through-channel (150) is arranged to hold bone graft material,
wherein a posterior portion of each of the first and second transverse plate members is movable closer to or farther from one another as an anterior portion of each of the first and second transverse plate members respectively move farther from or closer to one another.

12. The kit (300) for a spinal disc device (100, 100_{A}, 100_{B}) of claim 11, wherein each first transverse plate member (105) further comprises a first through-hole (125) and each second transverse plate member (110) further comprises a second through-hole (140), the first and second through-holes (125, 140) arranged to communicate with the central through-channel (150) when the first component (90) is coupled to the second component (95).

13. The kit (300) for a spinal disc device (100, 100_{A}, 100_{B}) of claim 11 or 12, wherein each first transverse plate member (105) of the plurality of first components (90) has a different lateral dimension, or a different lateral configuration, or a different longitudinal thickness; or wherein each second transverse plate member (110) of the plurality of second components (95) has a different lateral dimension, or a different lateral configuration, or a different longitudinal thickness.

14. The kit (300) for a spinal disc device (100, 100_{A}, 100_{B}) of claim 11, wherein for each of the first and second components, the first configuration of the first arcuate extension and the second configuration of the second arcuate extension (120) are each a partially spherical, convex shell configuration.

## Patentansprüche

1. Bandscheibenvorrichtung (100, 100_{A}, 100_{B}), die zwischen angrenzenden Wirbelkörpern (170, 175) implantierbar ist, Folgendes umfassend:
ein erstes querliegendes Plattenelement (105);
eine erste bogenförmige Verlängerung (115), die mit dem ersten querliegenden Plattenelement (105) verbunden ist und sich in Längsrichtung von diesem erstreckt, wobei die erste bogenförmige Verlängerung (115) eine erste Anordnung aufweist;
ein zweites querliegendes Plattenelement (110);
eine zweite bogenförmige Verlängerung (120), die mit dem zweiten querliegenden Plattenelement (110) verbunden ist und sich in Längsrichtung von diesem erstreckt, wobei die zweite bogenförmige Verlängerung (120) eine zweite, zur ersten Anordnung der ersten bogenförmigen Verlängerung (115) passende Anordnung aufweist, die in Bezug auf die erste bogenförmige Verlängerung (115) verschiebbar ist, wobei die erste bogenförmige Verlängerung (115) und die zweite bogenförmige Verlängerung (120) ferner so angeordnet sind, dass sie einen zentralen durchgehenden Kanal (150) zwischen dem ersten und dem zweiten querliegenden Plattenelement (105, 110) ausbilden; und
wobei der zentrale durchgehende Kanal (150) so angeordnet ist, dass er Knochentransplantationsmaterial aufnimmt,
wobei ein hinterer Abschnitt jeweils des ersten und des zweiten querliegenden Plattenelements näher zu einander oder weiter weg von einander bewegbar ist als sich ein vorderer Abschnitt jeweils des ersten bzw. des zweiten querliegenden Plattenelements weiter weg voneinander oder näher zu einander bewegt.

2. Bandscheibenvorrichtung (100, 100_{A}, 100_{B}) nach Anspruch 1, wobei das erste querliegende Plattenelement (105) ferner eine erste Durchgangsbohrung (125) aufweist und das zweite querliegende Plattenelement (110) ferner eine zweite Durchgangsbohrung (140) aufweist, wobei die erste und die zweite Durchgangsbohrung (125, 140) so angeordnet sind, dass sie mit dem zentralen durchgehenden Kanal (150) in Verbindung stehen.

3. Bandscheibenvorrichtung (100, 100_{A}, 100_{B}) nach Anspruch 1, wobei die erste Anordnung der ersten bogenförmigen Verlängerung (115) und die zweite Anordnung der zweiten bogenförmigen Verlängerung (120) jeweils eine teilweise kugelförmige, konvexe Schalenanordnung ist.

4. Bandscheibenvorrichtung (100, 100_{A}, 100_{B}) nach Anspruch 3, wobei die erste bogenförmige Verlängerung (115) sowohl in einer Sagittalebene als auch in einer Frontalebene in Bezug auf die zweite bogenförmige Verlängerung (120) verschiebbar ist.

5. Bandscheibenvorrichtung (100, 100_{A}, 100_{B}) nach einem der Ansprüche 1 bis 4, wobei eine Innenfläche (225) der ersten bogenförmigen Verlängerung (115) eine teilweise kugelförmige, konkave Anordnung aufweist, und eine Außenfläche der zweiten bogenförmigen Verlängerung (120) eine teilweise kugelförmige, konvexe Anordnung aufweist, oder wobei eine Innenfläche (235) der zweiten bogenförmigen Verlängerung (120) eine teilweise kugelförmige, konkave Anordnung aufweist und eine Außenfläche der ersten bogenförmigen Verlängerung (115) eine teilweise kugelförmige, konvexe Anordnung aufweist.

6. Bandscheibenvorrichtung (100, 100_{A}, 100_{B}) nach einem der Ansprüche 1 bis 5, wobei die erste bogenförmige Verlängerung (115) und die zweite bogenförmige Verlängerung (120) ferner so angeordnet sind, dass sie einander in Längsrichtung überlappen.

7. Bandscheibenvorrichtung (100, 100_{A}, 100_{B}) nach einem der Ansprüche 1 bis 6, ferner Folgendes umfassend:
wenigstens eine Verlängerungsendplatte, die in Längsrichtung mit dem ersten querliegenden Plattenelement (105) oder dem zweiten querliegenden Plattenelement (110) verbunden werden kann.

8. Bandscheibenvorrichtung (100, 100_{A}, 100_{B}) nach Anspruch 7, wobei die wenigstens eine Verlängerungsendplatte ferner eine dritte Durchgangsbohrung umfasst, die so angeordnet ist, dass sie mit dem zentralen durchgehenden Kanal (150) in Verbindung steht.

9. Implantierbare Bandscheibenvorrichtung (100, 100_{A}, 100_{B}) nach Anspruch 1, wobei ein oder mehrere des ersten querliegenden Plattenelements (105), des zweiten querliegenden Plattenelements (110), der ersten bogenförmigen Verlängerung (115) und der zweiten bogenförmigen Verlängerung (120) ein Material umfassen, das aus der Gruppe bestehend aus Pyrokohlenstoff, Titan, porösem Titan, Titannitrid, Tantal, Kobalt, Chrom, Polyethylen, Kohlefaser, PEEK® (Polyetheretherketon), Acetal-Homopolymerharz, Aluminiumoxid, Zirkonium, Siliziumkarbid, Siliziumnitrid, Edelstahl, Diamant oder einem diamantähnlichen Material gewählt wird.

10. Implantierbare Bandscheibenvorrichtung (100, 100_{A}, 100_{B}) nach Anspruch 1, wobei der zentrale durchgehende Kanal (150) ein erstes Volumen aufweist und die Bandscheibenvorrichtung ein zweites Volumen aufweist, wobei das erste Volumen wenigstens 50 % des zweiten Volumens beträgt.

11. Bausatz (300) für eine Bandscheibenvorrichtung (100, 100_{A}, 100_{B}), die zwischen angrenzenden Wirbelkörpern implantierbar ist, Folgendes umfassend:
eine Vielzahl erster Bauteile (90), wobei jedes Bauteil (90) aus der Vielzahl der ersten Bauteile (90) Folgendes umfasst:
ein erstes querliegendes Plattenelement (105); und
eine erste bogenförmige Verlängerung (115), die mit dem ersten querliegenden Plattenelement (105) verbunden ist und sich in Längsrichtung von diesem erstreckt, wobei die erste bogenförmige Verlängerung (115) eine erste Anordnung aufweist;
und
eine Vielzahl zweiter Bauteile (95), wobei ein zweites Bauteil aus der Vielzahl der zweiten Bauteile mit einem ersten Bauteil aus der Vielzahl der ersten Bauteile verbunden werden kann, um eine Bandscheibenvorrichtung auszubilden, wobei jedes zweite Bauteil (95) aus der Vielzahl der zweiten Bauteile (95) Folgendes umfasst:
ein zweites querliegendes Plattenelement (110); und
eine zweite bogenförmige Verlängerung (120), die mit dem zweiten querliegenden Plattenelement (110) verbunden ist und sich in Längsrichtung von diesem erstreckt, wobei die zweite bogenförmige Verlängerung (120) eine zweite, zur ersten Anordnung der ersten bogenförmigen Verlängerung (115) passende Anordnung aufweist, die in Bezug auf die erste bogenförmige Verlängerung (115) für jedes der ersten Bauteile (90) verschiebbar ist, wobei die erste bogenförmige Verlängerung (115) und die zweite bogenförmige Verlängerung (120) ferner so angeordnet sind, dass sie einen zentralen durchgehenden Kanal (150) zwischen dem ersten und dem zweiten querliegenden Plattenelement (105, 110) für jedes der ersten und zweiten Bauteile (90, 95) ausbilden; und
wobei der zentrale durchgehende Kanal (150) so angeordnet ist, dass er Knochentransplantationsmaterial aufnimmt,
wobei ein hinterer Abschnitt jeweils des ersten und des zweiten querliegenden Plattenelements näher zu einander oder weiter weg von einander bewegbar ist als sich ein vorderer Abschnitt jeweils des ersten bzw. des zweiten querliegenden Plattenelements weiter weg voneinander oder näher zu einander bewegt.

12. Bausatz (300) für eine Bandscheibenvorrichtung (100, 100_{A}, 100_{B}) nach Anspruch 11, wobei jedes erste querliegende Plattenelement (105) ferner eine erste Durchgangsbohrung (125) umfasst und das zweite querliegende Plattenelement (110) ferner eine zweite Durchgangsbohrung (140) umfasst, wobei die erste und die zweite Durchgangsbohrung (125, 140) so angeordnet sind, dass sie mit dem zentralen durchgehenden Kanal (150) in Verbindung stehen, wenn das erste Bauteil (90) mit dem zweiten Bauteil (95) verbunden ist.

13. Bausatz (300) für eine Bandscheibenvorrichtung (100, 100_{A}, 100_{B}) nach Anspruch 11 oder 12, wobei jedes erste querliegende Plattenelement (105) aus der Vielzahl der ersten Bauteile (90) eine unterschiedliche Querabmessung oder eine unterschiedliche laterale Anordnung oder eine unterschiedliche längsgerichtete Dicke aufweist; oder wobei jedes zweite querliegende Plattenelement (110) aus der Vielzahl der zweiten Bauteile (95) eine unterschiedliche Querabmessung oder eine unterschiedliche laterale Anordnung oder eine unterschiedliche längsgerichtete Dicke aufweist.

14. Bausatz (300) für eine Bandscheibenvorrichtung (100, 100_{A}, 100_{B}) nach Anspruch 11, wobei für jedes der ersten und zweiten Bauteile die erste Anordnung der ersten bogenförmigen Verlängerung und die zweite Anordnung der zweiten bogenförmigen Verlängerung (120) jeweils eine teilweise kugelförmige, konvexe Schalenanordnung ist.

## Revendications

1. Disque intervertébral (100, 100_{A}, 100_{B}) implantable entre des corps vertébraux adjacents (170, 175), comprenant :
un premier élément de plaque transversale (105) ;
une première extension arquée (115) couplée à et s'étendant longitudinalement depuis le premier élément de plaque transversale (105), la première extension arquée (115) ayant une première configuration ;
un second élément de plaque transversale (110) ;
une seconde extension arquée (120) couplée à et s'étendant longitudinalement depuis le second élément de plaque transversale (110), la seconde extension arquée (120) ayant une seconde configuration d'appariement avec la première configuration de la première extension arquée (115) et déplaçable de manière amovible par rapport à la première extension arquée (115), la première extension arquée (115) et la seconde extension arquée (120) étant en outre disposées pour former un canal central (150) entre les premier et second éléments de plaque transversale (105, 110) ; et
le canal central (150) étant disposé pour maintenir un matériau de greffe d'os,
une section postérieure de chacun des premier et second éléments de plaque transversale étant déplaçable plus proche ou plus loin l'un de l'autre comme une section antérieure de chacun des premier et second éléments de plaque transversale respectivement se déplaçant plus loin ou plus proche l'un de l'autre.

2. Disque intervertébral (100, 100_{A}, 100_{B}) selon la revendication 1, avec lequel le premier élément de plaque transversale (105) comprend en outre un premier trou (125) et le second élément de plaque transversale (110) comprend en outre un second trou (140), le premier et le second trous (125, 140) étant disposés pour communiquer avec le canal central (150).

3. Disque intervertébral (100, 100_{A}, 100_{B}) selon la revendication 1, avec lequel la première configuration de la première extension arquée (115) et la seconde configuration de la seconde extension arquée (120) sont chacune une configuration de coque convexe, partiellement sphérique.

4. Disque intervertébral (100, 100A, 100B) selon la revendication 3, avec lequel la première extension arquée (115) est déplaçable de manière amovible dans à la fois un plan sagittal et un plan coronal par rapport à la seconde extension arquée (120).

5. Disque intervertébral (100, 100_{A}, 100_{B}) selon l'une quelconque des revendications 1 à 4, avec lequel une surface interne (225) de la première extension arquée (115) a une configuration concave, partiellement sphérique et une surface externe de la seconde extension arquée (120) a une configuration convexe, partiellement sphérique, ou une surface interne (235) de la seconde extension arquée (120) a une configuration concave, partiellement sphérique et une surface externe de la première extension arquée (115) a une configuration convexe, partiellement sphérique.

6. Disque intervertébral (100, 100_{A}, 100_{B}) selon l'une quelconque des revendications 1 à 5, avec lequel la première extension arquée (115) et la seconde extension arquée (120) sont en outre disposées pour se chevaucher l'une l'autre dans la dimension longitudinale.

7. Disque intervertébral (100, 100_{A}, 100_{B}) selon l'une quelconque des revendications 1 à 6, comprenant en outre :
au moins une plaque d'extrémité d'extension que l'on peut coupler longitudinalement au premier élément de plaque transversale (105) ou au second élément de plaque transversale (110).

8. Disque intervertébral selon la revendication 7, avec lequel l'au moins une plaque d'extrémité d'extension comprend en outre un troisième trou disposé pour communiquer avec le canal central (150).

9. Disque intervertébral implantable (100, 100_{A}, 100_{B}) selon la revendication 1, avec lequel un ou plusieurs du premier élément de plaque transversale (105), du second élément de plaque transversale (110), de la première extension arquée (115) et de la seconde extension arquée (120) comprend/comprennent un matériau choisi dans le groupe constitué par : le carbone pyrolytique, le titane, le titane poreux, le nitrure de titane, le tantale, le cobalt, le chrome, le polyéthylène, la fibre de carbone, le PEEK® (polyéther éther cétone), une résine d'homopolymère d'acétal, l'alumine, l'oxyde de zirconium, le carbure de silicium, le nitrure de silicium, l'acier inoxydable, le diamant ou un matériau de type diamant.

10. Disque intervertébral implantable (100, 100_{A}, 100_{B}) selon la revendication 1, avec lequel le canal central (150) a un premier volume et le disque intervertébral a un second volume, le premier volume étant au moins 50 % du second volume.

11. Kit (300) pour un disque intervertébral (100, 100_{A}, 100_{B}) implantable entre des corps vertébraux adjacents, comprenant :
une pluralité de premiers composants (90), chaque premier composant (90) de la pluralité de premiers composants (90) comprenant :
un premier élément de plaque transversale (105) ; et
une première extension arquée (115) couplée à et s'étendant longitudinalement depuis le premier élément de plaque transversale (105), la première extension arquée (115) ayant une première configuration ; et
une pluralité de seconds composants (95), un second composant de la pluralité de seconds composants pouvant être couplé à un premier composant de la pluralité de premiers composants pour former un disque intervertébral, chaque second composant (95) de la pluralité de seconds composants (95) comprenant :
un second élément de plaque transversale (110) ;
une seconde extension arquée (120) couplée à et s'étendant longitudinalement depuis le second élément de plaque transversale (110), la seconde extension arquée (120) ayant une seconde configuration d'appariement avec la première configuration de la première extension arquée (115) et déplaçable de manière amovible par rapport à la première extension arquée (115) pour chacun des premiers composants (90), la première extension arquée (115) et la seconde extension arquée (120) étant en outre disposées pour former un canal central (150) entre les premier et second éléments de plaque transversale (105, 110) pour chacun des premier et second composants (90, 95) ; et
le canal central (150) étant disposé pour maintenir un matériau de greffe d'os,
une section postérieure de chacun des premier et second éléments de plaque transversale étant déplaçable plus proche ou plus loin l'un de l'autre comme une section antérieure de chacun des premier et second éléments de plaque transversale respectivement se déplaçant plus loin ou plus proche l'un de l'autre.

12. Kit (300) pour disque intervertébral (100, 100_{A}, 100_{B}) selon la revendication 11, avec lequel chaque premier élément de plaque transversale (105) comprend en outre un premier trou (125) et chaque second élément de plaque transversale (110) comprend en outre un second trou (140), le premier et le second trous (125, 140) étant disposés pour communiquer avec le canal central (150) quand le premier composant (90) est couplé au second composant (95).

13. Kit (300) pour disque intervertébral (100, 100_{A}, 100_{B}) selon la revendication 11 ou 12, avec lequel chaque premier élément de plaque transversale (105) de la pluralité de premiers composants (90) a une dimension latérale différente, ou une configuration latérale différente, ou une épaisseur longitudinale différente ; ou chaque second élément de plaque transversale (110) de la pluralité de seconds composants (95) ayant une dimension latérale différente, ou une configuration latérale différente, ou une épaisseur longitudinale différente.

14. Kit (300) pour disque intervertébral (100, 100_{A}, 100_{B}) selon la revendication 11, avec lequel pour chacun des premier et second composants, la première configuration de la première extension arquée et la seconde configuration de la seconde extension arquée (120) sont chacune une configuration de coque convexe, partiellement sphérique.
